# EUROPEAN PATENT SPECIFICATION

(11) **EP 1 454 602 B1**
(45) Date of publication and mention of the grant of the patent: **15.11.2006**
(21) Application number: 04251261.6
(22) Date of filing: 04.03.2004
(51) Int. Cl.: A61F 2/30, A61F 2/34, B29C 65/16

(54) **Method of improving the performance of a prosthetic bearing element**
Methode Verbesserung der Leistungsfähigkeit eines prothetischen Lagerelementes
Méthode d'améliorisation de la performance d'un élément de portée prothétique

(30) Priority: 05.03.2003 GB 0305021
(43) Date of publication of application: 08.09.2004
(73) Proprietor: HOWMEDICA INTERNATIONAL S. DE R.L., Shannon Co. Clare (IE)
(72) Inventor: Jones, Eric, Dr., Kidimo County Limerick (IE)
(74) Representative: Bridge-Butler, Alan James

(56) References cited:
- EP-A- 0 698 382
- US-A1- 2001 039 455
- HAENSCH D ET AL: "HARTE UND WEICHE KUNSTSTOFFE MIT DIODENLASER VERBINDEN. JOINING HARD AND SOFT PLASTICS WITH A DIODE LASER" KUNSTSTOFFE, CARL HANSER VERLAG. MUNCHEN, DE, vol. 88, no. 2, 1 February 1998 (1998-02-01), pages 210-212, XP000732887 ISSN: 0023-5563
- JONES I A ET AL: "USR OF INFRARED DYES FOR TRANSMISSION LASER WELDING OF PLASTICS" ANTEC. CONFERENCE PROCEEDINGS, XX, XX, 2000, pages 1166-1170, XP000952346
- POTENTE H ET AL: "LASER-DURCHSTRAHLSCHWEISSEN VON PE-HD" KUNSTSTOFFE, CARL HANSER VERLAG. MUNCHEN, DE, vol. 87, no. 3, 1 March 1997 (1997-03-01), pages 348-350, XP000703688 ISSN: 0023-5563

## Description

This invention relates to a method of improving the performance of a prosthetic bearing element and to elements made by such a process.

US 2001/039455 A1 refers to the use of laser light energy in the manufacture of a cartilage plug device and in which there are a plurality of anchoring elements extending from a continuous upper surface which are of different hardnesses which can be fastened together by treating the surfaces to be joined with a dark pigment so that laser light energy may be absorbed creating an intense heat and shock wave thereby fusing the interface together. The laser energy is used as a means of bonding or joining the interface together.

In the Article by Haensch, D. et al : *"HART UND WEICHE KUNSTSTOFF MIT DIODENLASER VERBINDED. JOINING HARD AND SOFT PLASTICS WITH A DIODE LASER", KUNSTSTOFFE, CARL HANSER VERLAG. MUNCHEN, DE. vol.88, No. 2. 1 February 1998 (1998-02-01), pages 210-212. AP000732887. ISSN: 0023-5563* it refers, again, to bonding plastics material together by using laser energy. It refers to allowing laser energy to pass through a laser transparent joint element and then be absorbed in a thin layer of a second joint element and converted into heat to obtain bonding. In an example it refers to a natural coloured thermoplastic (as the transparent component) being welded to a black coloured TPE (as the absorbent component). This article also therefore refers to methods of bonding two materials together.

EP 0 698 382 describes a prosthetic bearing element which comprises a backing supporting a bearing liner having a bearing surface.

The backing is said to be made from a rigid polymeric material which has a minimum hardness value of 65 N/mm² and the bearing liner is made from a "soft" elastomeric polyurethane material having a hardness value of 3.0 to 9.0 N/mm². The specification describes typical material which can be used for these bearings and a way of fabricating them by injection moulding. The particular construction allows a method for the attachment of this type of bearing element that may be held in a metal support if required and the construction is robust to reduce the amount of penetration and creep when the element is subjected to loads through the femoral component of either a hip or a knee. The bearing could also be used in other joints.

The injection moulding process employed in EP 0 698 382 was fully explored by the Applicants to describe the range of moulding conditions that were acceptable and as a result the present Applicants have now developed a process which provides improved properties.

According to the present invention a method of improving the performance of a prosthetic bearing element which comprises a backing made from a "rigid" polymeric bearing material which has a minimum hardness value of 65 N/mm² and which supports a bearing liner having a bearing surface and made from a "soft" elastomeric polyurethane material having a hardness value of 3.0 to 9.0 N/mm² (using machine testing method BS 2782; Pt3 method 365D) and bonding the backing to the liner is characterised in that the opacity of said bearing liner is arranged to allow the passage of a suitable laser beam through it and the opacity of said backing is arranged to prevent the passage of said laser beam which has been passed through said bearing liner, bonding the backing to the bearing liner and then treating the bearing liner and backing with said laser beam to cause improved fusion by laser welding.

Thus the technique can be applied to achieve improvement in interface properties of surface substrates bonded to the soft polyurethane bearing surface and a method of improving interfacial bonding of the system set forth in EP 0 698 328 in areas that are subjected to high stress levels.

The preferred method is to produce a composite material for the backing, from a hard PU and fibre such as carbon, Kevlar, glass or filler such as hydroxylapatite, barium sulphate, zirconia, although this list is not exclusive.

In a preferred method a hard (75D) commercial grade of polyurethane was filled with a carbon fibre.

A low power laser beam can be used to scan over the bearing surface. The energy passes directly through the bearing surface provided that the material is clear enough and is relatively unaffected, but is absorbed at the interface when it meets the darker composite layer of the backing. This energy is transformed into heat and results in local fusion. The overall result is that the interface bonding strength is increased.

The invention can be performed in various ways and some embodiments will now be described by way of example and with reference to the accompanying drawings in which :
Figure 1 shows the use of a composite backing on a PU bearing surface showing low frictional properties;
Figure 2 is a graph showing an estimation of fusion bond thickness measured by Fourier Transform Infra red Spectroscopy;
Figure 3 is a diagrammatic view of a prosthetic bearing cup showing examples of rupture of the fusion between backing and wear surface layers;
Figure 4 shows two graphs showing typical peel strength values; and,
Figure 5 is a graph showing the stability of laser assisted fusion bond after short-term ageing.

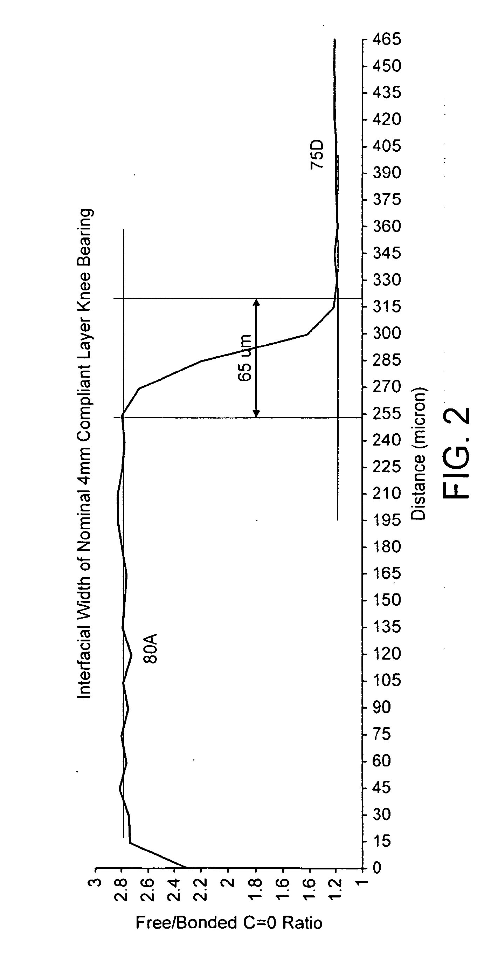

As referred to above the injection moulding process of EP 0 698 382 was fully explored to describe the range of moulding conditions that were acceptable. Of particular interest was the strength of the bond between the bearing and support layers. This interface is highly stressed during the various loading regimes encountered during the walking cycle, and is liable to fail unless maximum fusion between the two materials is achieved. The extent of this fusion was assessed by carrying out a detailed peel strength series of experiments using thicknesses equivalent to those of the actual bearing. Another method used was the estimation of the extent of this fusion layer using Fouier Transform Infra red Spectroscopy. As shown in Figure 2 the combination of the hard and soft polyurethane resin described gave the best peel strength values. Bonding of other polymers, including UHMWPE, composites and metals did not achieve interface strengths high enough to sustain the shear forces developed during simulated wear testing.

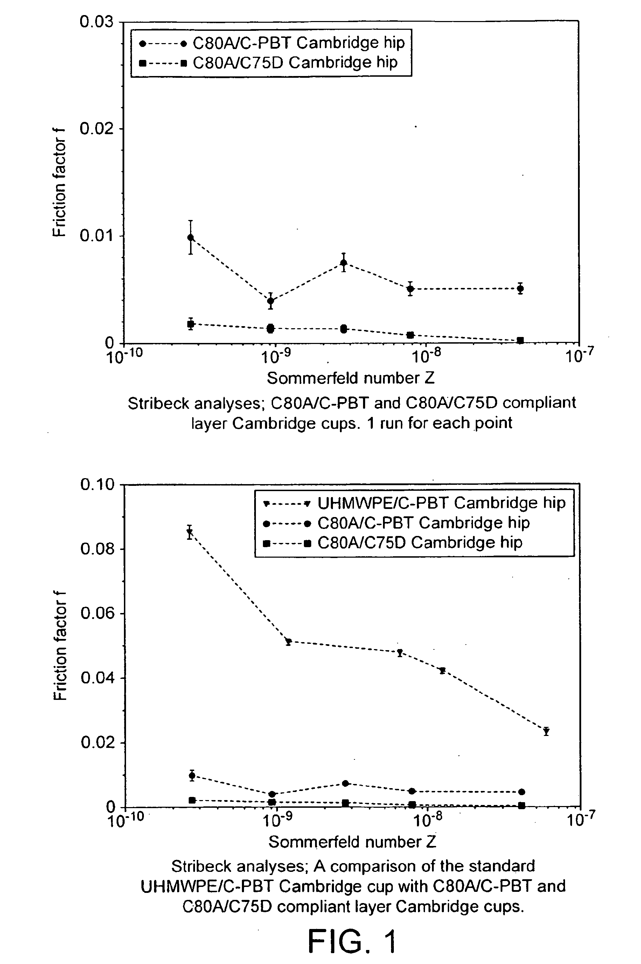

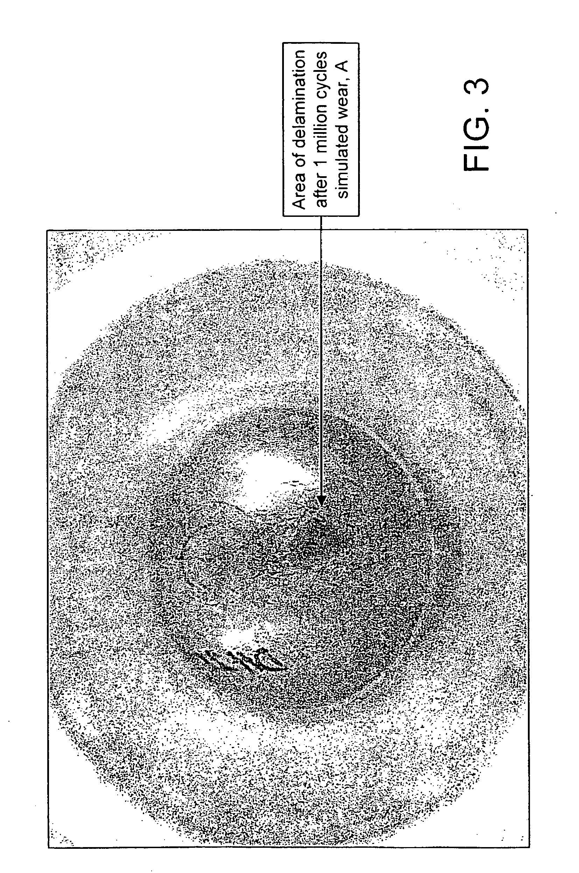

Auger et al. (DD Auger et al Proc Instn Mech\engrs Vol 209 1995 83-91) reported such findings in a series of simulated wear tests on knee bearings. Interfacial debonding occurred in many of the samples tested because the bonding between the bearing material (polyurethane) and the substrate (titanium) was insufficient. The Applicants carried out a series of tests using a composite substrate to a polyurethane bearing, and the interface failed quickly (less than 10,000 cycles. Figure 1 shows how the composite construct is able to function with superior performance, but only in the short term. In very rare instances the Applicants' preferred option failed when extreme testing conditions have been used. Figure 3 shows an example of such a failure seen in a knee bearing during wear simulated testing and using a misaligned bearing to give a worse case scenario. The area of delamination after 1 million cycles of simulated wear being indicated by reference letter A.

EP 0 698 382 refers to optimum bonding being approached using the hard and soft polyurethanes referred to. In certain instances however it may be preferred to have the backing a little stiffer such that the backing may be reduced in thickness, or the stiffer and harder material may reduce the extent of creep, or the water absorption may be reduced and other reasons also. However, when such composite materials are used in the interfacial bonding strength is compromised. Increasing the stiffness can be significantly effected either by changing the existing PU hard backing or inclusion of a stiffer medium.

The method according to the present invention can be applied to achieve improvement in interface properties of stiffer substrates bonded to the soft polyurethane bearing surface, and a method of improving the interfacial bonding of the method set forth in EP 0 698 382 (soft PU bearing and hard PU backing) in areas that are subjected to high stress levels.

A preferred method according to the present invention is to produce a composite material for the backing, from the hard PU and a fibre such as carbon, Kevlar, glass, or filler such as hydroxylapatite, barium sulphate, zirconia, although this list is not mutually exclusive.

### EXAMPLE 1

In a method to be investigated, hard polyurethane (Bionate 75D) was filled with carbon fibres (Solartrim) to the extent of 15%.

Melt compounding was used to mix in the carbon fibres that were of staple length 6-mm, prior to injection moulding. All compounding took place using a Prism TSE16 twin-screw extruder. The extruder had co-rotating twin screws with intermeshing flights. The screws were 16-mm in diameter with a compression ratio of 15:1 (length/diameter ratio). The extent of shear required was optimised by modifying the various screw configures. For the material studied, the extruder temperature profile was set at:

| | |
|---|---|
| Die | 220°C |
| Zone 4 | 220°C |
| Zone 3 | 210°C |
| Zone 2 | 205°C |
| Zone 1 | 195°C |

### And the torque set to 12.6 - 13.9 Nm (55%)

The material was hauled off at the rate of 3m/minute, and chopped into pellets of length suitable for injection moulding.

In order to test the invention, standard peel test samples were made as previously reported in EP 0 698 382. The following moulding conditions were employed to produce this backing using a standard 70T Arburg injection moulding machine:

| | |
|---|---|
| Temperature profile | 230, 230, 225, 210 ° C |
| Mould temperature | 30°C |
| Injection pressure | 50 bar |
| Holding pressure | 25 bar |
| Injection time | 10 secs |
| Hold time | 20 secs |
| Cooling time | 50 secs |
| Injection speed | fast |

This carbon fibre filled hard PU composite material now acts as the backing or support material, and a soft polyurethane (Bionate 80A) was injection moulding over one surface using the following conditions:

| | |
|---|---|
| Temperature profile | 220, 220, 215, 200°C |
| Mould temperature | 20°C |
| Injection pressure | 50 bar |
| Holding pressure | 25 bar |
| Injection time | 3 secs |
| Hold time | 10 secs |
| Cooling time | 20 secs |
| Injection speed | fast |

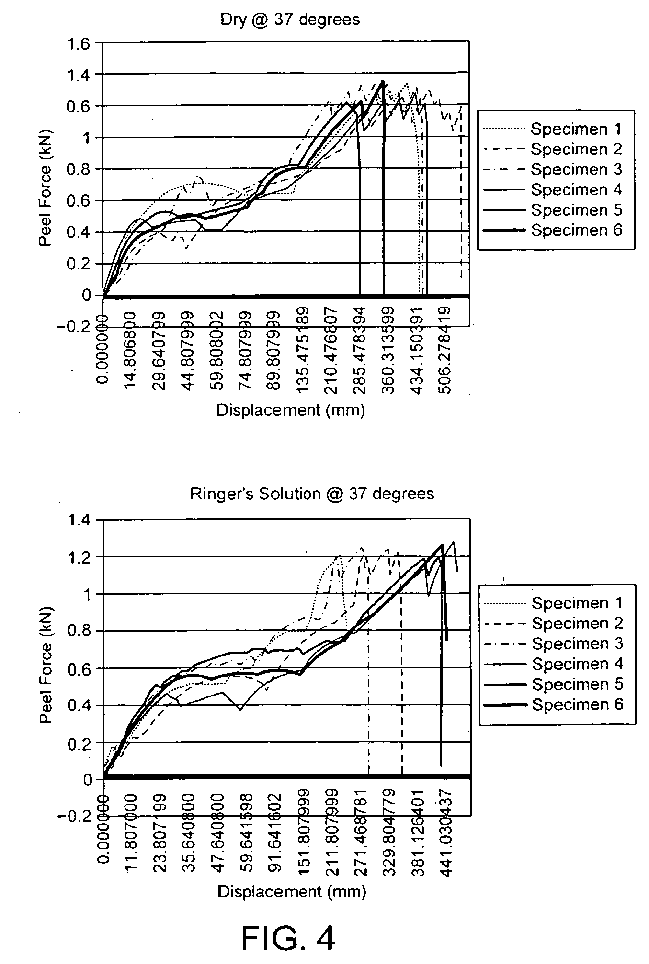

This produced a construction that permits other design options to be considered. The strength of the interface is, however, reduced in comparison to the all-pure PU system, and would therefore be likely to fail in service. However, a method of improving this interface has been developed using laser technology. A low power laser beam is scanned over the bearing surface. The energy passes directly through the clear layer relatively unaffected, but is absorbed at the interface when it meets the dark composite layer. This energy is transferred into heat and results in local fusion. It may be necessary to impart pressure to the device to ensure proper consolidation of the interface. The net result is that the interface bonding strength is increased to the level as recorded for the all pure PU system (see Figure 4). It will be appreciated that the capacity of the bearing liner is such as to allow the laser beam to pass through it but the backing opacity is sufficient to prevent passage of the beam.

A further composite backing construction was developed by alloying thermoplastic resins or thermoplastic composites with the PU materials. In a separate series of experiments, a composite was formed from carbon fibre reinforced polybutyleneterephthalate blended with the hard PU resin.

### EXAMPLE 2

The new composite materials for evaluation were a blends of Biothane 75D with 30% carbon fibre reinforced polybutyleneterephthalate at levels of 70/30, 80/20 and 90/10 % respectively. Prior to processing in the extruder as previously described, the blends were tumble mixed by shaking in a plastic container for 5 minutes.

For all three blends of material studied, the extruder temperature profile was set at:

| | |
|---|---|
| Die | 220°C |
| Zone 4 | 220°C |
| Zone 3 | 210°C |
| Zone 2 | 205°C |
| Zone 1 | 195°C |

And the torque set to 14.1-15.1 Nm (60%).

The material was hauled off at the rate of 3 - 4 m/minute, and chopped into pellets of length suitable for injection moulding.

In order to test the invention, standard peel test samples were made as previously reported in EP 0 698 382. The following moulding conditions were employed to produce this backing using a standard 70T Arburg injection moulding machine.

| | |
|---|---|
| Temperature profile | 230, 230, 225, 210°C |
| Mould temperature | 30°C |
| Injection pressure | 50 bar |
| Holding pressure | 25 bar |
| Injection time | 10 secs |
| Hold time | 20 secs |
| Cooling time | 50 secs |
| Injection speed | fast |

These carbon fibre filled hard PU composite materials now acts as the backing or support material, and a soft polyurethane (Bionate 80A) was injection moulding over one surface using the following conditions.

| | |
|---|---|
| Temperature profile | 225, 220, 215, 200°C |
| Mould temperature | 20°C |
| Injection pressure | 50 bar |
| Holding pressure | 25 bar |
| Injection time | 3 secs |
| Hold time | 10 secs |
| Cooling time | 20 secs |
| Injection speed | fast |

The fused interface was, however, reduced, with a subsequent reduction in the bonding strength, (to a lower level than in the previous example). This interfacial bond strength was, however, increased by using laser scanning in the method described above.

It is also possible to use just a thermoplastic composite as the backing material. This was tried as shown in Figure 1 when the interfacial bond strength was clearly inadequate. Although there is some type of adhesion between the composite backing and bearing surface, it is likely that these are attractive forces similar to Van der Waal forces of physical attraction. Such forces are weak, and are not able to sustain any prolonged activity under load. The bond can be improved again using the laser scanning process whereby heat is generated at the interface. The type of thermoplastic is of importance as this governs the conditions used and the extent of bonding.

In the Applicants' example, CFRPBT was used which has a melting point close to that of the PU, and bond strength was good. The task is more difficult as the melting point difference increases, so that the use of CFRPEEK is more problematic.

Biocompatible fillers can also be incorporated into the matrix of the PU to increase rigidity, and also impart further design possibilities. For example, hydroxylapatite was blended with the PU, which in addition to increasing the stiffness, also provided the possibility of increasing osteoactivity on the backing. Again, this process disimproved the interfacial bond strength, but this could be improved with laser scanning. In this case it may be necessary to add an energy adsorber in the form of an infra red sensing pigment either into the initial moulding compound or to the surface of one of the components prior to over-moulding. This approach is also valid for the two pure polymers if additional bond strength is required in areas of high stress.

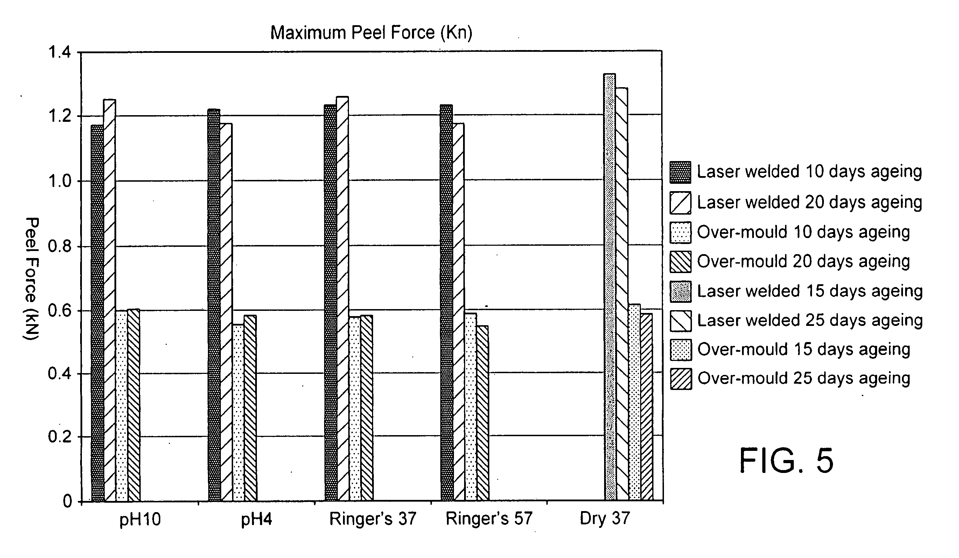

Improvements in interfacial bonding of these composite constructions are only of benefit if the effect is sustained in aqueous environment. A series of studies was effected on one of the samples to determine the stability of the interface. Figure 5 highlights these results and shows that at least over the short term the ageing is not significant.

The required relative opacities of the liner and the backing can be obtained by the use of appropriate fillers or as described above or infra red dyes, typically as referenced by Sevedenko MM "Determined Spectral characteristics of pigment absorption and scattering in the middle T/R spectral range". Optics and Spectroscopy Vol. 76,3, (1994) 418-420.

Diode or Nd:YAG laser welding methods were found to be the most suitable. Due to the composite carbon filler in the polyurethane composite material in the examples tested, this material has excellent laser absorbent properties, therefore the standard diode laser welding method was the most suited welding method to be employed. A very small test sample was welded with different laser welding techniques, in order to validate the literature findings. Results from these initial tests indicated that diode laser welding gave the strongest bond, without causing deformation of the specimen material.

Further analysis of the diode laser welding method on the sample specimen, revealed critical clamping forces, beam width, welding speed and welding power. A total of eight different welds were carried out, altering the critical parameters for each laser weld. It can be seen from the results of these eight trial welds, see Table 1, the impact of the critical welding parameters on the joint. Weld 1 was a focused weld, due to the beam width, overall joint strength was reasonable, but due to the intensity of the weld, burning of the polyurethane was found. Weld 2 had similar parameters to weld 1 but, due to the lower welding speed, more burning of the polyurethane material was found, thus a decrease in joint strength. When the beam width was increased in weld 3, the clamp pressure was also increased, this gave a stronger weld with less burning of the polyurethane.

Learning from the previous welds, clamp pressure was increased in weld 4, again less burning found and bond strength increased. Having increased the clamp pressure and reduced the laser power, optimum welds were found in welds 5 and 6. Other welding was carried out by increasing the laser power and welding speed proportionally. This was considered as a time saving exercise in welding, and therefore a reduced welding cost. However, results from welds 7 and 8, which were carried out at high speed and power, gave very poor weld and severe burning of the polyurethane. The sample laser welding test provided an excellent incite into the optimum parameters required in the welding of the main batch of specimen, which were aged and peel tested. Upon analysis of this sample weld, the main batch of specimens were welded at optimum laser welding conditions.

| No. | Beam width | Clamp pressure | Power | Welding speed | Results |
|---|---|---|---|---|---|
| | (mm) | (psi) | (W) | (mm/mm) | |
| 1 | 5 | 50 | 10 | 200 | Reasonable strength bond, burning |
| 2 | 5 | 50 | 10 | 180 | Poor bond strength, burning |
| 3 | 8 | 60 | 10 | 150 | Good weld, wide track |
| 4 | 8 | 70 | 10 | 150 | Good weld, strong bond |
| 5 | 8 | 80 | 9 | 140 | Excellent weld, excellent bond |
| 6 | 8 | 90 | 9 | 140 | Excellent weld excellent bond |
| 7 | 5 | 50 | 150 | 2500 | Burning, poor weld |
| 8 | 5 | 50 | 150 | 3000 | Burning, poor weld |

A total of five ageing procedures were selected for the welded specimen. A total of twenty eight specimens were welded at optimum laser welding conditions. Six of each specimen were assigned randomly to three of the ageing processes and five specimens assigned to two of the ageing processes. Due to the relatively short ageing time available, three of the ageing methods were accelerated ageing. The first set of specimens were stored in dry sealed conditions at room temperature, these specimens were comparison specimens, this allowed other aged specimens to be evaluated for ageing. Three of these specimens were peel tested 15 days after welding and three specimens 25 days after welding. The results of these peel tests were found in the following section.

Ageing at 37° C in Ringer's solution, was carried out in order to simulate human host conditions. These specimens were immersed in Ringer's solution, and placed into a temperature controlled covered water bath. These conditions gave a humid environment, where the Ringer's solution had very little evaporation and concentration was not altered. Alternatively a temperature controlled oven could have been employed, but significant evaporation of the Ringer's solution would have been experienced and concentration of the solution altered. Three specimens were removed from the Ringer's solution after 10 days and peel tested. The remaining specimens were removed from the Ringer's solution after 20 days and also peel tested. Results of the peel tests are described in the following section.

Accelerated ageing was carried out in three different methods. Accelerated aging at 57° C was carried out in Ringer's solution. Having increased the temperature by 20°C over the host environment temperature, ageing was accelerated by approximately four times the standard rate. This allowed the prediction of longer term ageing effects on the joint bond, in a relatively short period of time. The environment temperature was again maintained by employing a temperature controlled water bath. The water bath was again selected for the same benefits outlined of the ageing of specimen at 37° C in Ringer's solution. After a period of 10 days ageing in the described environment, three of the specimens were peel tested, due to the accelerated ageing this was the equivalent to 40 days ageing in the host environment conditions. The remaining three specimens were peel tested after 20 days of ageing, again due the acceleration this ageing period was equivalent to 80 days of ageing at the host environment conditions.

Due to the variations of acid and alkaline concentrations in the human body, due to diet, stress and other factors, extreme conditions were simulated in accelerated ageing, Five specimens were immersed in a phosphate buffered solution with a pH level of four. This would at least simulate the worse case acidic conditions in the regions of body fluids, where this material bond would be utilised in total joint replacements. The immersed specimens were placed in an enclosed temperature controlled water bath at 37° C. Peel testing was carried out on two of the specimen after 10 days ageing, the further three remaining specimens were peel tested after 20 days of ageing.

Finally, the fifth set of specimens were immersed in a phosphate buffered solution with a pH level of ten. After 10 days ageing in this environment, two specimens were peel tested. The remaining three specimens were peel tested after 20 days. Since body fluids in the region of total joint replacements can vary in pH concentration, both acid and alkaline pH levels were simulated. In normal situations the body fluid in total joint regions would vary from pH of 5 to 8, this depends on diet, stress, injury to the local tissue and many other factors. Therefore, the pH levels selected in these ageing processes are greater than those found in tissue body fluids.

## Claims

1. A method of improving the performance of a prosthetic bearing element which comprises a backing made from a "rigid" polymeric bearing material which has a minimum hardness value of 65 N/mm² and which supports a bearing liner having a bearing surface and made from a "soft" elastomeric polyurethane material having a hardness value of 3.0 to 9.0 N/mm² (using machine testing method BS 2782; Pt3 method 365D) and bonding the backing to the liner **characterised in that** the opacity of said bearing liner is arranged to allow the passage of a laser beam through it and the opacity of said backing is arranged to prevent the passage of said laser beam which has passed through said bearing liner, bonding the backing to the bearing liner and then treating the bearing liner and backing with said laser beam to cause improved fusion by laser welding.

2. A method as claimed in claim 1 **characterised in that** a composite material is produced for the backing which includes a hard polyurethane and a fibre or filler.

3. A method as claimed in claim 2 **characterised in that** said fibre a fibre is carbon, Kevlar, glass, and said filler is hydroxylapatite, barium sulphate, zirconia, or a combination thereof.

4. A method as claimed in claim 3 **characterised in that** said composite material is a hard (75D) commercial grade of polyurethane filled with a carbon fibre.

5. A method as claimed in claim 4 **characterised in that** the carbon fibre is of various fibre lengths and compositions.

6. A method as claimed in claims 2 to 5 **characterised in that** said composite backing material is formed by alloying thermoplastic resins or thermoplastic composites with the hard polyurethane material.

7. A method as claimed in claim 6 **characterised in that** said composite is formed from carbon fibre reinforced polybutyleneterephthalate blended with hard polyurethane resin.

8. A method as claimed in claims 2 to 5 **characterised in that** said backing material is a thermoplastic composite.

9. A method as claimed in claim 8 **characterised in that** CFRPBT is employed.

10. A method as claimed in any one of the preceding claims **characterised by** the use of fillers or infra red dyes to obtain the relative opacities of the bearing liner and backing.

11. A method as claimed in any one of the preceding claims **characterised in that** a diode or Nd:YAG laser welding method is used.

12. A method as claimed in any one of the preceding claims 1 to 7 **characterised by** imparting pressure to the bearing liner and backing to ensure proper consolidation of the interface.

13. A method as claimed in any one of the preceding claims **characterised in that** the prosthetic bearing element is an acetabular cup.

## Patentansprüche

1. Verfahren zur Verbesserung der Leistungsfähigkeit eines prothesentragenden Elementes, das einen Stützkörper umfaßt, der aus einem "rigiden" polymeren Trägermaterial mit einem minimalen Härtewert von 65 N/mm² ist, und der eine Trägerauskleidung mit einer Trägeroberfläche stützt, die aus einem "weichen" elastomeren Polyurethanmaterial ist und einen Härtewert von 3,0 bis 9,0 N/mm² aufweist (unter Verwendung des Maschinentestverfahrens BS 2782; Pt3, Verfahren 365D) und wobei der Stützkörper an die Auskleidung gebunden ist, **dadurch gekennzeichnet, daß** die Opazität der Trägerauskleidung so eingerichtet ist, daß ein Laserstrahl hindurchgehen kann, und die Opazität des Stützkörpers so eingerichtet ist, daß der Laserstrahl, der durch die Trägerauskleidung hindurchgegangen ist, nicht durch diesen gehen kann, und der Stützkörper an die Trägerauskleidung gebunden wird und dann die Trägerauskleidung und der Stützkörper mit dem Laserstrahl behandelt werden, um so die Verschmelzung durch Laserschweißen zu verbessern.

2. Verfahren nach Anspruch 1, **dadurch gekennzeichnet, daß** ein Verbundstoffmaterial für den Stützkörper hergestellt wird, das ein hartes Polyurethan und eine Faser oder einen Füllstoff umfaßt.

3. Verfahren nach Anspruch 2, **dadurch gekennzeichnet, daß** die Faser eine Faser aus Kohlenstoff, Kevlar, Glas ist, und das der Füllstoff Hydroxylapatit, Bariumsulfat, Zirconiumdioxid oder eine Kombination davon ist.

4. Verfahren nach Anspruch 3, **dadurch gekennzeichnet, daß** das Verbundstoffmaterial ein hartes (75D) handelsübliches Polyurethan, gefüllt mit einer Kohlenstoffaser ist.

5. Verfahren nach Anspruch 4, **dadurch gekennzeichnet, daß** die Kohlenstoffaser verschiedene Faserlängen und Zusammensetzungen aufweist.

6. Verfahren nach den Ansprüchen 2 bis 5, **dadurch gekennzeichnet, daß** das Verbundstoff-Stützkörpermaterial durch die Legierung thermoplastischer Harze oder thermoplastischer Verbundstoffe mit dem harten Polyurethanmaterial hergestellt wird.

7. Verfahren nach Anspruch 6, **dadurch gekennzeichnet, daß** der Verbundstoff aus Kohlenstoffaser-verstärktem Polybutylenterephthalat, gemischt mit hartem Polyurethanharz, gebildet wird.

8. Verfahren nach den Ansprüchen 2 bis 5, **dadurch gekennzeichnet, daß** das Stützkörpermaterial ein thermoplastischer Verbundstoff ist.

9. Verfahren nach Anspruch 8, **dadurch gekennzeichnet, daß** CFRPBT eingesetzt wird.

10. Verfahren nach einem der vorhergehenden Ansprüche, **gekennzeichnet durch** die Verwendung von Füllstoffen oder Infrarotfarbstoffen zum Erhalt der relativen Opazitäten der Trägerauskleidung und des Stützkörpers.

11. Verfahren nach einem der vorhergehenden Ansprüche, **dadurch gekennzeichnet, daß** ein Dioden- oder Nd:YAG-Laserschweißverfahren angewandt wird.

12. Verfahren nach einem der vorhergehenden Ansprüche 1 bis 7, **gekennzeichnet durch** das Ausüben von Druck auf die Trägerauskleidung und den Stützkörper, um so die richtige Verfestigung der Grenzfläche sicherzustellen.

13. Verfahren nach einem der vorhergehenden Ansprüche, **dadurch gekennzeichnet, daß** das prothesentragende Element eine Hüftgelenkpfanne ist.

## Revendications

1. Procédé d'amélioration de la performance d'un élément de palier prothétique qui comprend un renfort fabriqué en un matériau de palier polymère "rigide" qui a une valeur de dureté minimale de 65 N/mm² et qui supporte un revêtement de palier ayant une surface de palier et fabriqué en un matériau élastomère polyuréthane "mou", ayant une valeur de dureté de 3,0 à 9,0 N/mm² (en utilisant la méthode de test sur banc d'essai BS 2782 ; Pt3 méthode 365D) et la liaison du renfort au revêtement, **caractérisé en ce que** l'opacité dudit revêtement de palier est déterminée pour permettre le passage d'un rayon laser à travers lui et l'opacité dudit renfort est déterminée de manière à éviter le passage dudit rayon laser qui est passé à travers ledit revêtement de palier, et on relie le renfort au revêtement de palier puis on traite le revêtement de palier et le renfort avec ledit rayon laser pour provoquer une fusion améliorée par soudage au laser.

2. Procédé selon la revendication 1, **caractérisé en ce qu'**un matériau composite est produit pour le renfort, qui inclut un polyuréthane dur et une fibre ou une charge.

3. Procédé selon la revendication 2, **caractérisé en ce que** ladite fibre est une fibre de carbone, de Kevlar, de verre, et ladite charge est l'hydroxylapatite, le sulfate de baryum, la zircone ou une combinaison de ceux-ci.

4. Procédé selon la revendication 3, **caractérisé en ce que** ledit matériau composite est un polyuréthane de qualité commerciale dur (75 D), chargé d'une fibre de carbone.

5. Procédé selon la revendication 4, **caractérisé en ce que** la fibre de carbone est faite en diverses longueurs et compositions de fibre.

6. Procédé selon les revendications 2 à 5, **caractérisé en ce que** ledit matériau de renfort composite est formé par alliage de résines thermoplastiques ou de composites thermoplastiques avec le matériau polyuréthane dur.

7. Procédé selon la revendication 6, **caractérisé en ce que** ledit composite est formé d'un polybutylène téréphtalate renforcé de fibre de carbone mélangé avec de la résine polyuréthane dure.

8. Procédé selon les revendications 2 à 5, **caractérisé en ce que** ledit matériau de renfort est un composite thermoplastique.

9. Procédé selon la revendication 8, **caractérisé en ce que** du CFRPBT est utilisé.

10. Procédé selon l'une quelconque des revendications précédentes, **caractérisé par** l'utilisation de charges ou de colorants infrarouge pour obtenir les opacités relatives du revêtement de palier et du renfort.

11. Procédé selon l'une quelconque des revendications précédentes, **caractérisé en ce qu'**une méthode de soudage à la diode ou au laser Nd:YAG est utilisée.

12. Procédé selon l'une quelconque des revendications précédentes 1 à 7, **caractérisé par** l'application d'une pression sur le revêtement de palier et le renfort pour garantir une consolidation adéquate de l'interface.

13. Procédé selon l'une quelconque des revendications précédentes, **caractérisé en ce que** l'élément de palier prothétique est une cupule cotyloïdienne.
